Europäisches Patentamt

European Patent Office

Office européen des brevets

(11) Veröffentlichungsnummer : **0 345 586 B1**

(12) # EUROPÄISCHE PATENTSCHRIFT

(45) Veröffentlichungstag der Patentschrift :
**01.07.92 Patentblatt 92/27**

(51) Int. Cl.$^5$ : **B01F 17/00**

(21) Anmeldenummer : **89109632.3**

(22) Anmeldetag : **29.05.89**

(54) **Verfahren zur Herstellung stabiler, niedrigviskoser Öl-in-Wasser-Emulsion polarer Ölkomponenten.**

Verbunden mit 89906080.0/0419505
(europäische
Anmeldenummer/Veröffentlichungsnummer)
durch Entscheidung vom 27.06.91.

(30) Priorität : **06.06.88 DE 3819193**

(43) Veröffentlichungstag der Anmeldung :
**13.12.89 Patentblatt 89/50**

(45) Bekanntmachung des Hinweises auf die
Patenterteilung :
**01.07.92 Patentblatt 92/27**

(84) Benannte Vertragsstaaten :
**AT BE CH DE ES FR GB IT LI NL SE**

(56) Entgegenhaltungen :
**ENCYCLOPEDIA OF EMULSION TECHNO-
LOGY, Band I, Editie P-Bedrev, 1983, Seiten
337-367, M. Decker N.V.**

(56) Entgegenhaltungen :
**THE J. AMER. OIL CHEM. SOC., Band 33, Nr. 8,
August 1956, Seiten 364-367; R.R. BENERITO
et al.: "Fat emulsion. Effect of heaton solubility of hydrophilic emulsifiers"
TENSIDE, Band 21, Nr. 1, Januar/Februar 1984,
Seiten 28-33, München, DE; "Standardisierte
Stabilitäts-Charakterisierung vonEmulsionen"**

(73) Patentinhaber : **Henkel
Kommanditgesellschaft auf Aktien
Postfach 1100 Henkelstrasse 67
W-4000 Düsseldorf-Holthausen (DE)**

(72) Erfinder : **Schambil, Fred, Dr.
Niederstrasse 96
W-4019 Monheim (DE)**
Erfinder : **Striepling, Gert-Lothar
Am Dammsteg 62
W-4000 Düsseldorf 13 (DE)**
Erfinder : **Tesmann, Holger, Dr.
Vennstrasse 61
W-4000 Düsseldorf 12 (DE)**

## Beschreibung

Die Erfindung betrifft ein Verfahren zur Herstellung von Öl-in-Wasser-Emulsionen polarer Ölkomponenten mit einer oder mehreren Esterfunktionen im Molekül oder von Mischungen solcher polarer Öle mit kleineren Mengen unpolarer Kohlenwasserstofföle unter Bedingungen, die zu besonders stabilen und niedrigviskosen Emulsionen führen.

Es ist bekannt, daß Öl-in-Wasser-Emulsionen, die mit nichtionogenen Emulgatoren hergestellt und stabilisiert sind, beim Erwärmen eine Phaseninversion erleiden, d. h., daß bei höheren Temperaturen die äußere, wäßrige Phase zur inneren Phase werden kann. Dieser Vorgang ist in der Regel reversibel, d. h., daß sich beim Abkühlen wieder der ursprüngliche Emulsionstyp zurückbildet. Es ist auch bekannt, daß die Lage der Phaseninversionstemperatur von vielen Faktoren abhängig ist, z. B. von der Art und dem Phasenvolumen der Ölkomponente, von der Hydrophilie und der Struktur des Emulgators oder der Zusammensetzung des Emulgatorsystems, vgl. z. B. K. Shinoda und H. Kunieda in Encyclopedia of Emulsion Technology, Vol. I, ed. P. Becher 1983 (M. Decker, N.Y.), S. 337 - 367. Weiterhin ist es bekannt, daß Emulsionen, die bei oder wenig unterhalb der Phaseninversionstemperatur hergestellt werden, sich durch besondere Feinteiligkeit und Stabilität auszeichnen, während solche, die oberhalb der Phaseninversionstemperatur hergestellt werden, weniger feinteilig sind, (vgl. S. Friberg, C. Solans, J. Colloid Interface Sci,, 66, 367 - 368 (1978)).

F. Schambil, F. Jost und M. J. Schwuger berichten in "Progress in Colloid & Polymer Science $\underline{73}$, (1987), 37 - 47 über die Eigenschaften kosmetischer Emulsionen, die Fettalkohole und Fettalkoholpolyglycolether enthalten und beschreiben dabei auch, daß Emulsionen, die oberhalb der Phaseninversionstemperatur hergestellt wurden, eine niedrigere Viskosität und eine hohe Lagerstabilität aufweisen.

In den genannten Druckschriften wurden jedoch nur Emulsionen untersucht, deren Ölphase ganz oder überwiegend aus unpolaren Kohlenwasserstoffen besteht. Dem gegenüber weisen entsprechende Emulsionen, deren Ölkomponente ganz oder überwiegend aus polaren Estern und Triglyceridölen besteht, bei Temperaturen unterhalb 100 °C bei Verwendung der bekannten Emulgatoren oder Emulgatorkombinationen keine Phaseninversion auf.

Es bestand daher die Aufgabe für ganz oder überwiegend polare Ölkomponenten ein geeignetes Emulgatorsystem zu finden, das es gestattet, Emulsionen zu erzeugen, die bei Temperaturen unter 100 °C invertieren und die auf diese Weise in besonders stabile und niedrigviskose Emulsionen überführt werden können.

Gegenstand der Erfindung ist ein Verfahren zur Herstellung niedrigviskoser Öl-in-Wasser-Emulsionen von Ölkomponenten (A), die aus

50 - 100 Gew.-% Mono- und/oder Diestern der Formeln (I) $R^1COOR^2$ , (II) $R^2OOC-R^3-COOR^2$ und (III) $R^1COO-R^3-OOCR^1$ worin $R^1$ und $R^2$ Alkylgruppen mit 1 - 22 C-Atomen oder Alkenylgruppen mit 8 - 22 C-Atomen und $R^3$ Alkylengruppen mit 2 - 16 C-Atomen sind und die mindestens 10 C-Atome enthalten und gegebenenfalls 0 - 50 Gew.-% Fettsäuretriglyceriden von Fettsäuren mit 8 - 22 C-Atomen und gegebenenfalls 0 - 25 Gew.-% eines Kohlenwasserstofföls bestehen, indem man diese mit

(B) 0,1 bis 0,5 Gewichtsteilen, bezogen auf 1 Gewichtsteil der Ölkomponente, eines Emulgators vom Typ der Ethylenoxid-Anlagerungsprodukte an Fettalkohole mit 16 - 22 C-Atomen oder an Partialester von Polyolen mit 3 - 6 C-Atomen und Fettsäuren mit 14 - 22 C-Atomen, mit einem HLB-Wert von 11 - 12 und bevorzugt zusätzlich

(C) 0,1 - 0,5 Gewichtsteilen, bezogen auf 1 Gewichtsteil der Ölkomponente, eines Coemulgators vom Typ der gesättigten Fettalkohole mit 16 - 22 C-Atomen oder der Partialester von Polyolen mit 3 - 6 C-Atomen und gesättigten Fettsäuren mit 14 - 22 C-Atomen
bei einer Temperatur oberhalb des Schmelzpunktes des Gemisches aus Ölkomponente (A), Emulgator (B) und Coemulgator (C) mit mindestens 1 Gewichtsteil Wasser, bezogen auf 1 Gewichtsteil der Ölkomponente, emulgiert und die Emulsion auf eine Temperatur innerhalb oder oberhalb des Phaseninversions-Temperaturbereichs erhitzt - oder die Emulsion bei dieser Temperatur herstellt - und dann die Emulsion auf eine Temperatur unterhalb des Phaseninversions-Temperaturbereichs abkühlt und gegebenenfalls mit Wasser weiter verdünnt.

Innerhalb der oben definierten Zusammensetzungen und unter den genannten Arbeitsbedingungen zeigen Emulsionen der polaren Ölkomponente vom Typ der genannten Mono- und Diester eine Phaseninversion unterhalb 100 °C, so daß sich unter praktischen Bedingungen nach dem angegebenen Verfahren auch mit diesen polaren Ölkomponenten besonders stabile, feinteilige und niedrigviskose Emulsionen herstellen lassen.

Ölkomponenten vom Typ der Mono- und Diester der Formeln I, II und III sind als kosmetische und pharmazeutische Ölkomponenten sowie als Gleit- und Schmiermittelkomponenten bekannt. Unter den Mono- und Diestern dieser Art kommt den bei Raumtemperatur (20 °C) flüssigen Produkten die größte Bedeutung zu. Als Ölkomponenten geeignete Monoester (I) sind z. B. die Methylester und Isopropylester von Fettsäuren mit 12 - 22 C-Atomen, wie z. B. Methyllaurat, Methylstearat, Methyloleat, Methylerucat, Isopropylpalmitat, Isopropylmyristat, Isopropylpalmitat, Isopropylstearat, Isopropyloleat. Andere Geeignete Monoester sind z. B. n-Butylstearat, n-Hexyllaurat, n-Decyloleat, Isooctylstearat, Isononylpalmitat, Isononyl-isononanoat, 2-Ethylhexyl-palmitat, 2-Ethylhexyl-laurat, 2-Hexyldecyl-steart, 2-Octyldodecyl-palmitat, Oleyloleat, Oleylerucat, Erucyloleat sowie Ester, die aus technischen aliphatischen Alkoholgemischen und technischen, aliphatischen Carbonsäuren erhältlich sind, z. B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z. B. im Jojobaöl oder im Spermöl vorliegen.

Geeignete Dicarbonsäureester (II) sind z. B. Di-n-butyl-adipat, Di-n-butyl-sebacat, Di-(2-ethylhexyl)-adipat, Di-(2-hexyldecyl)-succinat und Di-isotridecyl-acelaat. Geeignete Diolester (III) sind z. B. Ethylenglycol-dioleat, Ethylenglycol-di-isotridecanoat, Propylenglycol-di-(2-ethylhexanoat), Butandiol-di-isostearat und Neopentylglycol-di-caprylat.

Als Fettsäuretriglyceride können natürliche, pflanzliche Öle, z. B. Olivenöl, Sonnenblumenöl, Sojaöl, Erdnußöl, Rapsöl, Mandelöl, Palmöl, aber auch die flüssigen Anteile des Kokosöls oder des Palmkernöls sowie tierische Öle, wie z. B. Klauenöl, die flüssigen Anteile des Rindertalges oder auch synthetische Triglyceride, wie sie durch Veresterung von Glycerin mit Fettsäuren mit 8 - 22 C-Atomen erhalten werden, z. B. Triglyceride von Caprylsäure-Caprinsäure-Gemischen, Triglyceride aus technischer Ölsäure oder aus Palmitinsäure-Ölsäure-Gemischen.

Wie oben ausgeführt, eignen sich bevorzugt solche Mono- und Diester und Triglyceride als Ölkomponenten für das erfindungsgemäße Verfahren, die bei Normaltemperatur von 20 °C flüssig sind, es können aber auch höherschmelzende Fette und Ester, die den angegebenen Formeln entsprechen, in solchen Mengen mitverwendet werden, daß die Mischung der Ölkomponenten bei Normaltemperatur flüssig bleibt.

Die Ölkomponente kann auch Kohlenwasserstofföle in untergeordneten Mengen bis zu maximal 25 Gew.-%, bezogen auf die Ölkomponente, enthalten. Geeignete Kohlenwasserstoffe sind vor allem Paraffinöle und synthetisch hergestellte Kohlenwasserstoffe, z. B. flüssige Polyolefine oder definierte Kohlenwasserstoffe, z. B. Alkylcyclohexane, wie z. B. das 1.3-Di-isooctylcyclohexan.

Als Emulgatoren (B) geeignete nichtionogene Ethylenoxidanlagerungsprodukte an Fettalkohole mit 16 - 22 C-Atomen sind handelsüblich. Die technischen Produkte stellen Gemische homologer Polyglycolether der Ausgangsfettalkohle dar, deren mittlerer Oxethylierungsgrad der angelagerten Molmenge an Ethylenoxid entspricht. Als Emulgatoren können auch Ethylenoxidanlagerungsprodukte an Partialester aus einem Polyol mit 3 - 6 C-Atomen und Fettsäuren mit 14 - 22 C-Atomen verwendet werden. Solche Produkte werden z. B. durch Ethoxylierung von Glycerinmonostearat, Glycerinmonopalmitat, oder von Mono- und Di-Fettsäureestern des Sorbitans, z. B. von Sorbitanmonostearat oder Sorbitansesquioleat hergestellt. Die für das erfindungsgemäße Verfahren geeigneten Emulgatoren sollen einen HLB-Wert von 11 bis 12 aufweisen. Unter dem HLB-Wert (Hydrophil-Lipophil-Balance) soll ein Wert verstanden werden, der errechnet werden kann gemäß

$$HLB = \frac{100 - L}{5}$$

worin L der Gewichtsanteil der lipophilen Gruppen, d. h. der Fettalkyl- oder Fettacylgruppen in Prozent in den Ethylenoxidanlagerungsprodukten ist.

Bevorzugt geeignet als Emulgatoren sind Anlagerungsprodukte von 8 - 12 Mol Ethylenoxid an gesättigte Fettalkohole mit 16 - 22 C-Atomen. Zur erfindungsgemäßen Emulgierung von Ölkomponenten, die keine unpolaren Kohlenwasserstofföle enthalten, die also aus 50 - 100 Gew.-% Mono- und Diestern der Formeln I, II und III und 0 - 50 Gew.-% Fettsäuretriglyceriden bestehen, eignen sich als Emulgatoren insbesondere Anlagerungsprodukte von 8 - 12 Mol Ethylenoxid an einen gesättigten Fettalkohol mit 20 - 22 C-Atomen.

Zusätzlich zum Emulgator ist in vielen Fällen ein Coemulgator (C) zur Herstellung der Öl-in-Wasser-Emulsionen nach dem erfindungsgemäßen Verfahren erforderlich. Der Coemulgator ist aufgrund seiner Hydrophilie selbst nicht zur Herstellung von Öl-in-Wasser-Emulsionen geeignet, gemeinsam mit den oben definierten Emulgatoren lassen sich jedoch besonders stabile und feinteilige Emulsionen der polaren Ölkomponenten herstellen. Als Coemulgatoren sind erfindungsgemäß solche vom Typ der gesättigten Fettalkohole mit 16 - 22 C-Atomen, z. B. Cetylalkohol, Stearylalkohol, Arachidylalkohol oder Behenylalkohol oder Gemische dieser Alkohole geeignet, wie sie bei der technischen Hydrierung von pflanzlichen und tierischen Fettsäuren mit 16 - 22 C-Atomen oder der entsprechenden Fettsäuremethylester erhalten werden. Weiterhin eignen sich als Coemulgatoren Partialester aus einem Polyol mit 3 - 6 C-Atomen und gesättigten Fettsäuren mit 14 - 22 C-Atomen. Solche Partialester sind z. B. die Monoglyceride von Palmitin- und/oder Stearinsäure, die Sorbitanmono- und/oder -diester von Myristinsäure, Palmitinsäure, Stearinsäure oder von Mischungen dieser Fettsäuren, die Monoester aus Trimethylolpropan, Erythrit oder Pentaerythrit und gesättigten Fettsäuren mit 14 - 22 C-Atomen. Als Monoester werden auch die technischen Monoester verstanden, die durch Veresterung von 1 Mol Polyol mit 1 Mol Fettsäure erhalten werden und die ein Gemisch aus Monoester, Diester und unverestertem Polyol darstellen.

Besonders gut eignen sich für das erfindungsgemäße Verfahren als Coemulgatoren Cetylalkohol, Stearylalkohol oder ein Glycerin-, Sorbitan- oder Trimethylolpropan-Monoester einer gesättigten Fettsäure mit 14 - 22 C-Atomen oder Gemische dieser Stoffe.

Um nach dem erfindungsgemäßen Verfahren und mit den oben spezifizierten Ölkomponenten (A), Emulgatoren (B) und Coemulgatoren (C) besonders niedrigviskose Emulsionen zu erhalten, ist es erforderlich, diese Komponenten in näher definierten Mengenverhältnissen einzusetzen. Besonders geeignet ist ein Gewichtsverhältnis von A : B : C = 1 : 0,1 - 0,3 : 0,1 - 0,3 und bevorzugt wird ein Gewichtsverhältnis von A : B : C = 1 : 0,2 : 0,15 eingesetzt.

Das erfindungsgemäße Verfahren kann in der Weise durchgeführt werden, daß zunächst die Phaseninversionstemperatur bestimmt wird, indem man eine Probe der auf übliche Weise hergestellten Emulsion unter Verwendung eines Leitfähigkeitsmeßgerätes erhitzt und die Temperatur bestimmt, bei der die Leitfähigkeit stark abnimmt. Die Abnahme der spezifischen Leitfähigkeit der zunächst vorhandenen Öl-in-Wasser-Emulsion nimmt üblicherweise über einen Temperaturbereich von 2 - 8 °C von anfänglich über 1 Millisiemens pro cm (mS/cm) auf Werte unter 0,1 mS/cm ab. Dieser Temperaturbereich wird hier als Phaseninversions-Temperaturbereich bezeichnet.

Nachdem der Phaseninversions-Temperaturbereich bekannt ist, kann man das erfindungsgemäße Verfahren entweder in der Weise durchführen, daß man die zunächst wie üblich hergestellte Emulsion nachträglich auf eine Temperatur erhitzt, die innerhalb oder oberhalb des Phaseninversions-Temperaturbereichs liegt, oder in der Weise, daß man bereits bei der Herstellung der Emulsion eine Temperatur wählt, die innerhalb oder oberhalb des Phaseninversions-Temperaturbereichs liegt.

Die nach dem erfindungsgemäßen Verfahren hergestellten Öl-in-Wasser-Emulsionen weisen eine hohe Feinteiligkeit und Stabilität auf. Besonders auffallend ist, daß die erfindungsgemäß hergestellten Emulsionen eine wesentlich niedrigere Viskosität aufweisen als Emulsionen, die nach dem herkömmlichen Verfahren hergestellt wurden.

Durch das erfindungsgemäße Verfahren ist daher eine Möglichkeit geschaffen worden, auch Emulsionen von polaren Ölkomponenten in so niedriger Viskosität und mit deutlich erhöhter Stabilität herzustellen, wie dies bisher nur mit unpolaren Kohlenwasserstoffen möglich war.

Öl-in-Wasser-Emulsionen, wie sie nach dem erfindungsgemäßen Verfahren erhalten werden, finden Anwendung z. B. als Haut- und Körperpflegemittel, als Kühlschmiermittel oder als Textil- und Faserhilfsmittel. Besonders bevorzugt ist das erfindungsgemäße Verfahren zur Herstellung emulsionsförmiger Zubereitungen für die Haut- und Haarbehandlung geeignet. Die folgenden Beispiele sollen den Erfindungsgegenstand näher erläutern, ohne ihn hierauf zu beschränken.

<u>Beispiele</u>

1. Herstellung der Emulsionen (übliche Arbeitsweise)

Die Ölkomponenten, Emulgatoren und Coemulgatoren wurden gemischt und auf eine Temperatur oberhalb des Schmelzpunktes der Mischung erwärmt und homogenisiert. Dann wurde die Schmelze unter Rühren in das Wasser, welches auf etwa die gleiche Temperatur erhitzt war, einemulgiert. Die Zusammensetzung der Emulsionen ist der Tabelle I zu entnehmen.

2. Ermittlung der Phaseninversionstemperatur

Unter Verwendung einer Leitfähigkeitsmeßbrücke (Fa. Radiometer, Kopenhagen) wurde die elektrische Leitfähigkeit der Emulsionen in Abhängigkeit von der Temperatur ermittelt. Zu diesem Zweck wurde die Emulsion zunächst auf + 20 °C abgekühlt. Bei dieser Temperatur zeigten die Emulsionen eine Leitfähigkeit von über 1 Millisiemens pro cm (mS/cm), d. h. sie lagen als Öl-in-Wasser-Emulsionen vor. Durch langsames Erwärmen mit einer Heizrate von ca. 0,5 °C/min, die mit Hilfe eines Temperatur-Programmgebers in Verbindung mit einem Kryostaten gesteuert wurde, wurde ein Leitfähigkeitsdiagramm erstellt. Der Temperaturbereich, innerhalb welchem die Leitfähigkeit auf Werte unterhalb 0,1 mS/cm abfiel, wurde als Phaseninversions-Temperaturbereich notiert. Bei allen in Tabelle I aufgeführten Emulsionen lag dieser Temperaturbereich unter 100 °C (Tabelle II, Phaseninversion).

3. Erfindungsgemäße Herstellung der Emulsionen

Die Emulsionen wurden, wie unter 1. beschrieben hergestellt und dann kurzzeitig (ca. 1 Minute) auf eine Temperatur im Phaseninversions-Temperaturbereich oder wenig darüber erhitzt (Tabelle II, Herstelltemperatur). Dann wurden die Emulsionen rasch, d. h. mit einer Abkühlrate von ca. 2 °C pro Minute, unter Rühren auf Raumtemperatur abgekühlt. Nach 1 Stunde Lagerung bei 20 °C wurde die Viskosität unter Verwendung eines Rotations-Viskorimeters (Brookfield, Type LVT) gemessen.

4. Vergleichsversuche

Zum Vergleich wurden die Emulsionen wie unter 1. beschrieben hergestellt, dabei aber nur auf eine Temperatur unterhalb des Phaseninversions-Temperaturbereichs erwärmt (Tabelle II, Herstelltemperatur). Die Abkühlung erfolgte analog wie unter 3. angegeben.

5. Ergebnis

Die Ergebnisse der erfindungsgemäßen Herstellung und der Vergleichsversuche sind für die Rezepturen gemäß Tabelle I in Tabelle II dargestellt. Bei erfindungsgemäßer Herstellung nach dem unter 3. angegebenen Verfahren wurden stets dünnflüssigere und stabile Öl-in-Wasser-Emulsionen erhalten. Die Vergleichsversuche hingegen ergaben Öl-in-Wasser-Emulsionen höherer Viskosität und - in einigen Fällen - von geringerer Stabilität.

EP 0 345 586 B1

## Tabelle I

| Zusammensetzung (Gew.-%) | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Isopropylmyristat | – | – | – | – | – | – | – | – | – | – | – | – | 20 | 20 | – | 20 |
| Isooctylstearat | – | – | – | – | – | – | – | – | – | – | 15 | – | – | – | – | – |
| Isononylpalmitat-stearat | – | – | – | – | – | – | – | – | – | 15 | – | – | – | – | – | – |
| Decyloleat | 15 | 20 | 20 | 20 | 15 | 15 | 10 | 15 | – | – | – | – | 15 | – | 15 | – |
| Oleyloleat | – | – | – | – | – | – | – | 20 | – | – | – | – | – | – | – | – |
| Di-n-butyladipat | – | – | – | – | – | – | – | – | – | – | – | 15 | – | – | – | – |
| Mandelöl | – | – | – | – | – | – | 10 | 5 | – | – | – | – | – | – | – | – |
| 1,3-Diisooctyl-cyclohexan | 5 | – | – | – | 5 | 5 | – | – | – | – | – | – | – | – | – | – |
| Paraffinöl (DAB 8) dickfl. | – | – | – | – | – | – | – | – | – | 5 | 5 | 5 | 5 | – | 5 | – |
| Cetyl-stearylakohol + 12 EO*) | 4 | – | – | – | – | – | – | – | – | – | 4 | 4 | 4 | 4 | 4 | 4 |
| Stearylalkohol + 8 EO*) | – | 4 | – | – | – | – | – | – | – | – | – | – | – | – | – | – |
| Arachidylalkohol + 10 EO*) | – | – | 4 | – | 4 | – | 4 | 4 | – | – | – | – | – | – | – | – |
| Behenylalkohol + 10 EO*) | – | – | – | 4 | – | 4 | – | – | 4 | 4 | – | – | – | – | – | – |

*) Anlagerungsprodukte von 12 bzw. 8 bzw. Mol Ethylenoxid an 1 Mol des angegebenen Alkohols

EP 0 345 586 B1

## Tabelle I (Fortsetzung)

| Zusammensetzung (Gew.-%) | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| Cetyl-stearylalkohol | 3 | 3 | 3 | 3 | 3 | 3 | 3 | 3 | 3 | 3 | 3 | 3 | – | – | – | – |
| Sorbitanmonolaurat | – | – | – | – | – | – | – | – | – | – | – | – | 3 | – | – | 3 |
| Sorbitanmonostearat | – | – | – | – | – | – | – | – | – | – | – | – | – | 3 | – | – |
| Trimethylolpropanmonomyristat | – | – | – | – | – | – | – | – | – | – | – | – | – | – | 3 | – |
| Wasser | 73 | 73 | 73 | 73 | 73 | 73 | 73 | 73 | 73 | 73 | 73 | 73 | 73 | 73 | 73 | 73 |

EP 0 345 586 B1

## Tabelle II

| Beispiel Nr. | 1 | | 2 | | 3 | | 4 | | 5 | |
|---|---|---|---|---|---|---|---|---|---|---|
| Phaseninversion °C | 93 – 99 | | 72,5 – 77,5 | | 81 – 85 | | 73 – 75,5 | | 72 – 75 | |
| Herstelltemperatur °C | 80 | 95 | 65 | 80 | 75 | 90 | 70 | 80 | 65 | 75 |
| Viskosität (mPas) 20 °C | 16400 | 20 | 1080 | 8 | 680 | 7 | 290 | 134 | 2040 | 35 |

| Beispiel Nr. | 6 | | 7 | | 8 | | 9 | | 10 | |
|---|---|---|---|---|---|---|---|---|---|---|
| Phaseninversion °C | 72 – 75 | | 83 – 88 | | 72 – 74 | | 75 – 84 | | 73,5 – 95 | |
| Herstelltemperatur °C | 65 | 75 | 80 | 90 | 65 | 75 | 70 | 90 | 70 | 95 |
| Viskosität (mPas) 20 °C | 1680 | 35 | 1680 | 230 | 1280 | 195 | 920 | 110 | 5500 | 1450 |

## Tabelle II (Fortsetzung)

| Beispiel Nr. | 11 | | 12 | | 13 | | 14 | | 15 | | 16 | |
|---|---|---|---|---|---|---|---|---|---|---|---|---|
| Phaseninversion °C | 94,5 – 99 | | 83,5 – 88 | | 80 – 88,5 | | 71 – 78 | | 65,5 – 71 | | 72 – 77 | |
| Herstelltemperatur °C | 85 | 99 | 80 | 95 | 75 | 95 | 70 | 95 | 55 | 75 | 70 | 85 |
| Viskosität (mPas) 20 °C | 4000 | 1200 | S* | 1050 | 10000 | 1,5 | S* | 3 | S* | 3 | 4000 | 55 |

S* Separation, Emulsion instabil

EP 0 345 586 B1

EP 0 345 586 B1

## Patentansprüche

1. Verfahren zur Herstellung niedrigviskoser Öl-in-Wasser-Emulsionen von Ölkomponenten (A), die aus 50 - 100 Gew.-% Mono- und/oder Diestern der Formeln

$$(I) \qquad R^1\text{-}COOR^2$$
$$(II) \qquad R^2OOC\text{-}R^3\text{-}COOR^2 \text{ und}$$
$$(III) \qquad R^1\text{-}COOR^3OOC\text{-}R^1$$

worin $R^1$ und $R^2$ Alkylgruppen mit 1 - 22 C-Atomen oder Alkenylgruppen mit 8 - 22 C-Atomen und $R^3$ Alkylengruppen mit 2 - 16 C-Atomen sind und die mindestens 10 C-Atome enthalten und gegebenenfalls aus 0 - 50 Gew.-% Fettsäuretriglyceriden von Fettsäuren mit 8 - 22 C-Atomen und gegebenenfalls 0 - 25 Gew.-% eines Kohlenwasserstofföls bestehen, dadurch gekennzeichnet, daß man diese mit

(B) 0,1 bis 0,5 Gewichtsteilen, bezogen auf 1 Gewichtsteil der Ölkomponente, eines Emulgators vom Typ der Ethylenoxidanlagerungsprodukte an Fettalkohole mit 16 - 22 C-Atomen oder an Partialester von Polyolen mit 3 - 6 C-Atomen und Fettsäuren mit 14 - 22 C-Atomen, mit einem HLB-Wert von 11 - 12 und bevorzugt zusätzlich

(C) 0,1 bis 0,5 Gewichtsteilen, bezogen auf 1 Gewichtsteil der Ölkomponente, eines Coemulgators vom Typ der gesättigten Fettalkohole mit 16 - 22 C-Atomen oder der Partialester von Polyolen mit 3 - 6 C-Atomen und gesättigten Fettsäuren mit 14 - 22 C-Atomen

bei einer Temperatur oberhalb des Schmelzpunktes des Gemisches aus Ölkomponente (A), Emulgator (B) und Coemulgator (C) mit mindestens 1 Gewichtsteil Wasser, bezogen auf 1 Gewichtsteil der Ölkomponente, emulgiert und die Emulsion auf eine Temperatur innerhalb oder oberhalb des Phaseninversions-Temperaturbereiches erhitzt - oder die Emulsion bei dieser Temperatur herstellt - und dann die Emulsion auf eine Temperatur unterhalb des Phaseninversions-Temperaturbereiches abkühlt und gegebenenfalls mit Wasser weiter verdünnt.

2. Verfahren nach Anspruch 1, dadurch gekennzeichnet, daß als Emulgator (B) ein Anlagerungsprodukt von 8 - 12 Mol Ethylenoxid an einen gesättigten Fettalkohol mit 16 - 22 C-Atomen eingesetzt wird.

3. Verfahren nach einem der Ansprüche 1 oder 2, dadurch gekennzeichnet, daß die Ölkomponente (A) aus 50 - 100 Gew.-% Mono- und/oder Diestern der Formeln I, II und III gegebenenfalls 0 - 50 Gew.-% Fettsäuretriglyceriden besteht und als Emulgator (B) ein Anlagerungsprodukt von 8 - 12 Mol Ethylenoxid an einen gesättigten Fettalkohol mit 20 - 22 C-Atomen eingesetzt wird.

4. Verfahren nach Anspruch 1 - 3, dadurch gekennzeichnet, daß als Coemulgator (C), Cetylalkohol, Stearylalkohol oder ein Glycerin-, Sorbitan- oder Trimethylolpropan-Monoester einer gesättigten Fettsäure mit 14 - 22 C-Atomen oder Gemische dieser Stoffe eingesetzt wird.

5. Verfahren nach Anspruch 1 - 4, dadurch gekennzeichnet, daß Ölkomponente (A), Emulgator (B) und Coemulgator (C) im Gewichtsverhältnis A : B : C = 1 : 0,1 - 0,3 : 0,1 - 0,3 und bevorzugt im Gewichtsverhältnis A : B : C = 1 : 0,2 : 0,15 eingesetzt werden.

## Claims

1. A process for the production of low-viscosity oil-in-water emulsions of oil components (A) consisting of 50 to 100% by weight monoesters and/or diesters corresponding to the following formulae

$$(I) \qquad R^1\text{-}COOR^2$$
$$(II) \qquad R^2OOC\text{-}R^3\text{-}COOR^2 \text{ and}$$
$$(III) \qquad R^1\text{-}COOR^3OOC\text{-}R^1$$

in which $R^1$ and $R^2$ are 1-22 C-alkyl groups or 8-22 C-alkenyl groups and $R^3$ represents 2-16 C-alkylene groups and which contain at least 10 carbon atoms, and optionally

0 to 50% by weight fatty acid triglycerides of 8-22 C-fatty acids and optionally

0 to 25% by weight of a hydrocarbon oil, characterized in that the oil components are emulsified with

(B) 0.1 to 0.5 part by weight - per part by weight of the oil component - of an emulsifier selected from the group of ethylene oxide adducts with 16-22 C-fatty alcohols or with partial esters of 3-6 C-polyols and 14-22 C-fatty acids and having an HLB value of 11 to 12, and preferably also with

(C) 0.1 to 0.5 part by weight - per part by weight of the oil component - of a co-emulsifier selected from the group of saturated 16-22 C-fatty alcohols or the partial esters of 3-6 C-polyols and saturated 14-22 C-fatty acids at a temperature above the melting point of the mixture of oil component (A), emulsifier (B) and co-emulsifier (C) containing at least 1 part by weight water per part by weight of the oil component and the emulsion

10

is heated to, or is prepared at, a temperature within or above the phase inversion temperature range, after which the emulsion is cooled to a temperature below the phase inversion temperature range and, optionally, further diluted with water.

2. A process as claimed in claim 1, characterized in that an adduct of 8 to 12 mol ethylene oxide with a saturated 16-22 C-fatty alcohol is used as the emulsifier (B).

3. A process as claimed in claim 1 or 2, characterized in that the oil component (A) consists of 50 to 100% by weight monoesters and/or diesters corresponding to formulae I, II and III and optionally
0 to 50% by weight fatty acid triglycerides and in that an adduct of 8 to 12 mol ethylene oxide with a saturated 20-22 C-fatty alcohol is used as the emulsifier (B).

4. A process as claimed in claims 1 to 3, characterized in that cetyl alcohol, stearyl alcohol or a glycerol, sorbitan or trimethylol propane monoester of a saturated 14-22 C-fatty acid or mixtures thereof is/are used as the coemulsifier (C).

5. A process as claimed in claims 1 to 4, characterized in that the oil component (A), the emulsifier (B) and the co-emulsifier (C) are used in a ratio by weight of A to B to C of 1 : 0.1 - 0.3 : 0.1 - 0.3 and preferably in a ratio by weight of A to B to C of 1 : 0.2 : 0.15.


## Revendications

1. Procédé de préparation d'émulsions huile dans l'eau peu visqueuses de composants huileux (A), qui sont constituées de:
50 à 100 % en poids de mono- et/ou de diesters des formules

$$(I) \quad R^1COOR^2$$
$$(II) \quad R^2OOC\text{-}R^3\text{-}COOR^2 \text{ et}$$
$$(III) \quad R^1COO\text{-}R^3\text{-}OOCR^1$$

dans lesquelles $R^1$ et $R^2$ représentent des groupements alkyle comportant 1 à 22 atomes de C, ou des groupement alcényle comportant 8 à 22 atomes de C, et où $R^3$ correspond à des groupements alkylène comportant 2 à 16 atomes de C, et qui renferment au moins 10 atomes de C et, le cas échéant, de
0 à 50 % en poids de triglycérides d'acides gras comportant 8 à 22 atomes de C et, le cas échéant, de
0 à 25 % en poids d'une huile hydrocarburée, caractérisé en ce que l'on émulsionne ceux-ci avec:
(B) 0,1 à 0,5 parties en poids, pour 1 partie en poids du composant huileux, d'un émulsifiant du type des produits d'addition de l'oxyde d'éthylène à des alcools gras comportant 16 à 22 atomes C ou à des esters partiels de polyols comprenant 3 à 6 atomes de C et à des acides gras comportant 14 à 22 atomes de C, avec une valeur HLB de 11 à 12 et, de préférence, en plus
(C) 0,1 à 0,5 parties en poids, pour 1 partie en poids du composant huileux, d'un co-émulsifiant du type des alcools gras saturés comportant 16 à 22 atomes de C ou des esters partiels de polyols comprenant 3 à 6 atomes de C et des acides gras saturés comprenant 14 à 22 atomes de C.
à une température située au-delà du point de fusion du mélange du composant huileux (A), de l'émulsifiant (B) et du co-émulsifiant (C) avec au moins 1 partie en poids d'eau pour 1 partie en poids du composant huileux, et en ce que l'on chauffe l'émulsion à une température située dans les limites ou au-delà de la plage de températures d'inversion de phase (ou en préparant l'émulsion à cette température), puis en refroidissant l'émulsion à une température située en-deçà de la plage de températures d'inversion de phase, et en diluant éventuellement davantage à l'eau.

2. Procédé selon la revendication 1, caractérisé en ce que l'on met en oeuvre comme émulsifiant (B) un produit d'addition de 8 à 12 moles d'oxyde d'éthylène à un alcool gras saturé comportant 16 à 22 atomes de C.

3. Procédé selon l'une des revendications 1 ou 2, caractérisé en ce que le composant huileux (A) est constitué de 50 à 100 % en poids de mono- et/ou de diesters des formules 1, II et III, le cas échéant,
à 50 % en poids de triglycérides d'acides gras et en ce que l'on met en oeuvre comme émulsifiant (B), un produit d'addition de 8 à 12 moles d'oxyde d'éthylène à un alcool gras saturé comportant 20 à 22 atomes de C.

4. Procédé selon les revendications 1 à 3, caractérisé en ce que l'on met en oeuvre comme co-émulsifiant (C), de l'alcool cétylique, de l'alcool stéarylique ou un mononoster de glycérine, de sorbitane ou de triméthylolpropane d'un acide gras saturé comportant 14 à 22 atomes de C ou de mélanges de ces substances.

5. Procédé selon les revendications 1 à 4, caractérisé en ce que le composant huileux (A), l'émulsifiant (B) et le co-émulsifiant (C) sont mis en oeuvre dans un rapport pondéral de A:B:C = 1:0,1 à 0,3:0,1 à 0,3 et, de préférence, dans un rapport pondéral de A:B:C = 1:0,2:0,15.